# EUROPÄISCHE PATENTSCHRIFT

(11) **EP 1 596 938 B1**
(45) Veröffentlichungstag und Bekanntmachung des Hinweises auf die Patenterteilung: **19.10.2011**
(21) Anmeldenummer: 04704615.6
(22) Anmeldetag: 23.01.2004
(51) Int. Cl.: A61N 2/02

(54) **ANORDNUNG ZUR DURCHFÜHRUNG EINER MAGNETFELDTHERAPIE UND VERFAHREN ZUR STROMBEREITSTELLUNG FÜR EINE MAGNETFELDTHERAPIE**
ASSEMBLY FOR CARRYING OUT MAGNETOTHERAPY AND METHOD FOR PROVIDING A CURRENT FOR MAGNETOTHERAPY
DISPOSITIF POUR METTRE EN OEUVRE UNE MAGNETOTHERAPIE ET PROCEDE POUR PREVOIR UN COURANT POUR UNE MAGNETOTHERAPIE

(30) Priorität: 31.01.2003 DE 10304085
(43) Veröffentlichungstag der Anmeldung: 23.11.2005
(73) Patentinhaber: vita-life International Franchising & Licensing AG, 9490 Vaduz (LI)
(72) Erfinder: HILBURG, Andreas, 46045 Oberhausen (DE)
(74) Vertreter: Freischem, Stephan
(86) Internationale Anmeldenummer: PCT/EP2004/050043
(87) Internationale Veröffentlichungsnummer: WO 2004/067090

(56) Entgegenhaltungen:
- EP-B- 0 594 655
- DE-A- 3 933 521
- GB-A- 2 295 093

## Beschreibung

Die Erfindung betrifft eine Anordnung zur Durchführung einer Magnetfeldtherapie gemäß dem Anspruch 1 sowie ein Verfahren zur Bereitstellung eines zeitlich variierenden Stroms durch ein Steuergerät gemäß dem Anspruch 13.

Magnetfeldtherapien, bei denen ein Organismus, insbesondere ein menschlicher Organismus, zur Steigerung des Wohlbefindens und zur Entspannung mit einem zeitlich variierenden Magnetfeld beaufschlagt wird, erfreuen sich steigender Beliebtheit. Der Patient wird über einen Applikator mit dem Magnetfeld beaufschlagt. Der Applikator weist elektrisch Leiterbahnen auf, welche von einem Strom durchflossen werden und auf diese weise das Magnetfeld erzeugen. Üblicherweise sind die Leiterbahnen des Applikators in einer Matte integriert, auf die sich der zu behandelnde Patient legt.

Es hat sich herausgestellt, daß bestimmte niederfrequent gepulste elektrisch Ströme ein über den Applikator auf den Patienten wirkendes, gepulstes Magnetfeld erzeugen, welches je nach den Parametern des Stromverlaufs und damit des Verlaufs der magnetischen Feldstärke unterschiedliche Wirkungen auf den Organismus des Patienten ausüben. Eine bestimmte Impulsform, welche eine gezielte Beeinflussung der Ionenkonzentration in beliebigen Körperbereichen hervorrufen soll, ist beispielsweise in dem europäischen Patent EP 0 594 655 B1 beschrieben.

Herkömmliche Magnetfeldtherapiegeräte erzeugen einen manuell durch einen Bediener eingestellten Impulsverlauf, der unabhängig von der tatsächlichen Wirkung der Magnetfeldtherapie und dem persönlichen Befinden des Patienten abläuft.

In dem europäischen Patent EP 0 729 318 ist eine Einrichtung zur Ermittlung der Wirkung gepulster Magnetfelder auf einen Organismus beschrieben, bei der um die Spule zur Erzeugung eines primären Magnetfeldes eine Antennenspule oder Meßspule angeordnet ist, die Sekundär-Feldsignale erfaßt, welche jeweils nach einem Impuls des primären Energiefeldes durch das im Organismus entstehende sekundäre und abklingende Magnetfeld in die Meßspule induziert werden. Mit dieser Vorrichtung läßt sich die Wirkung des Therapiegerätes, nämlich die Intensität des in dem Organismus erzeugten Magnetfeldes bestimmen. Indes läßt sich nicht das Resultat dieser Wirkung, d.h. die Beeinflussung des menschlichen Organismus durch die Beaufschlagung mit dem Magnetfeld bestimmen.

Es wurde auch vorgeschlagen, an das Steuergerät einen Biosensor anzuschließen, der eine vegetative oder motorische Funktion des Lebewesens erfaßt. Die genannte Druckschrift EP 594 655 B1 beschreibt z.B. allgemein den Einsatz einer Biofeedback Regelung zur Einstellung der optimalen Feldparameter des Magnetfeldes. Dabei wird in einer Ausführungsform zur Regelgrößengewinnung ein Pulsmeßgerät verwendet. Es wird in der Beschreibung angeführt, daß damit die Erkenntnis ausgewertet wird, daß bei optimaler Wirkung der gepulsten elektromagnetischen Felder sich der Pulsschlag verlangsamt. Die Druckschrift GB-A-2295033 offenbart eine Anordnung gemäß dem Oberbegriff des Anspruchs 1 und ein Verfahren gemäß dem Oberbegriff des Anspruchs 13.

Die Herzfrequenz hat sich jedoch als wenig aussagekräftig in bezug auf die Wirkung der Magnetfeldtherapie erwiesen. Ihre absolute Größe sowie das Maß ihrer Änderung ist vornehmlich von den physischen Merkmalen und der physischen Belastbarkeit des therapierten Lebewesens und nur in geringem Maße von der Wirkung der Magnetfeldtherapie abhängig.

Aufgabe der vorliegenden Erfindung ist es, eine Magnetfeldtherapieanordnung und ein Verfahren zur Bereitstellung eines zeitlich variierenden Stroms durch ein Steuergerät für eine derartige Therapieanordnung zu schaffen, bei denen während der Therapie der Zustand des therapierten Patienten zuverlässig erfaßt und berücksichtigt wird.

Diese Aufgabe wird erfindungsgemäß in bezug auf die Anordnung durch die Gesamtheit der Merkmale des Patentanspruchs 1 und in bezug auf das Verfahren durch die Gesamtheit der Merkmale des Patentanspruchs 13 gelöst.

Die durch den Pulssensor erfaßten Meßsignale werden einer Regelanordnung zugeführt, welche einen oder mehrere Parameter des Magnetfeldes in Abhängigkeit von den Meßsignalen einstellt. Die Regelanordnung ist bei einer praktischen Ausführungsform im Steuergerät angeordnet. Der Pulssensor erfaßt den Puls des Patienten. Der Herzschlag und somit der Puls ist einer der wesentlichen Bioparameter eines menschlichen oder tierischen Organismus. Wie weiter unten ausgeführt, lassen sich dem Pulsmeßsignal wertvolle Erkenntnisse über die Befindlichkeit des Patienten entnehmen.

Aus der periodischen Signalkurve des Pulssensors wird gemäß der Erfindung die Herzratenvariabilität ermittelt. Die Herzratenvariabilität ist ein Maß für die Änderung des Herzzyklus. Der Herzzyklus ist der Abstand zwischen zwei aufeinanderfolgenden Herzschlägen. Bei gesunden Menschen fluktuiert die Herzfrequenz, die in Ruhe zwischen 60 und 100 Sehlägen pro Minute liegt, in Abhängigkeit von der Atmung notmalenrweise um 15% und mehr. Die Herzfrequensäaderüngen sind das Ergebnis vieler miteinander in Verbindung stehender Regelkreise im Körper, die physiologische Schwankungen kompensieren. Die Herzfrequensänderung wird auch Herzratenvariabilität genannt und ist ein Maß für die allgemeine Anpassungsfähigkeit eines Organismus auf interne und externe Reize. Sie eignet sich sehr gut zur Beurteilung des aktuelle physiologischen Zustandes des therapierten Lebewesens sowie des Einflusses der Therapie auf diesen Zustand. Eine detailliertere Beschreibung der Ermittlung und Auswertung der Herzratenvariabilität folgt weiter unten.

Bei der erfindungsgemäßen Anordnung können bei Bedarf weitere Biosensoren wie beispielsweise Meßelektroden, Temperatursensoren, Widerstandssensoren, Atemmeßgeräte, Atemgasanalysegeräte verwendet werden. Durch diese Sensoren können beispielsweise folgende Bioparameter aufgenommen werden: Blutdruck, Sauerstoffsättigung des Blutes, Aktionspotentiale des Herzens (Elektrokardiogramm), Potentialschwankungen des Gehirns (Elektroenzephalogramm), Hauttemperatur, Hautwiderstand, Atemfrequenz, Atemvolumen, Atemgaszusammensetzung.

Da verschiedene Parameter des Magnetfeldes und somit des dem Applikationsmittel zugeführten Stroms die Befindlichkeit des Patienten beeinflussen, erlaubt die Pulsmessung und Analyse zur Ermittlung der Herzratenvariabilität eine erfolgsabhängige Regelung der Magnetfeldtherapie. Ferner werden bei der Einstellung der Parameter des Magnetfeldes nicht nur die unmittelbaren Wirkungen des Magnetfeldapplikators, nämlich die Stärke des aufgebrachten Magnetfeldes, berücksichtigt, sondern auch andere Einflüsse auf den Zustand und die Befindlichkeit des Patienten, welche von der Magnetfeldtherapie unabhängig sein können, aus dem Meßsignal des Pulssensors jedoch abgelesen werden können.

Wie beim Stand der Technik umfaßt der Applikator der erfindungsgemäßen Anordnung üblicherweise einen elektrischen Leiter, der mit einem Strom beaufschlagt wird. Bei einer praktischen Ausführungsform beeinflußt das Steuergerät einen oder mehrere Parameter des Stromsignals, mit welchem durch den Applikator das Magnetfeld erzeugt wird. Die beeinflußten Parameter sind beispielsweise die Dauer eines einzelnen Stromimpulses, die Frequenz der Wiederholung der einzelnen Stromimpulse innerhalb einer Gruppe periodischer Stromimpulse, die Pause, d.h. der Zeitabstand zwischen zwei aufeinanderfolgenden Gruppen von Stromimpulsen, deren Kehrwert auch als "Burst Frequenz" bezeichnet wird, die dem Leiter zugeführte Stromstärke und Stromspannung. Welcher Signalverlauf und welcher sich daraus ergebender Magnetfeldstärkenverlauf eine gewünschte Wirkung auf den Patienten hat, ist in der Literatur zur Magnetfeldtherapie ausführlich behandelt. Ein Beispiel hierfür ist die oben genannte EP 0 594 655 B1. Durch die aus dem Meßsignal des Pulssensors gewonnenen Informationen können die Signalpararneter derart variiert werden, das jeweils das erwünschte und aufgrund des Meßsignals sinnvolle Therapieergebnis erzielt wird.

Der Sensor zur Erfassung des Herzrhythmus, d.h. des Herzschlages oder Pulses des Patienten, ist bei einer praktischen Ausführungsform ein bekannter Puls-Oximetersensor. Die Pulsoximetrie ist ein Verfahren zur Bestimmung des Sauerstoffgehaltes (O₂-Gehaltes) des Blutes. Dabei wird ein photometrisches Meßverfahren eingesetzt. In Abhängigkeit davon, ob in das Hämoglobin des Blutes Sauerstoff gebunden ist (Oxihämoglobin), ändert sich die Farbe des Blutes. Stark sauerstoffhaltiges Blut hat eine rötliche Färbung, wogegen desoxigeniertes Blut seine Farbe zu einem blauen Farbton hin verändert. Ein Oximeter mißt die Farbänderung des Blutes. Dabei wird durch eine Lichtquelle des Oximeters ein Abschnitt des Körpers des Patienten, in dem Blutgefäße vorhanden sind, mit Licht durchstrahlt. Durch den Herzschlag und den mit dem Herzschlag variierenden Blutdruck ändert sich die Ausdehnung der Gefäße. Diese rhythmische Ausdehnung und Kontraktion der Gefäße führt zu einem im Rhythmus des Herzschlages verlaufenden Signal. Der pulsierende, d.h. variable Teil des erfaßten Signals ist auf das in den Arterien fließende Blut zurückzuführen, so daß der statische Teil des Meßsignals subtrahiert werden kann und über den variablen Teil die Farbbestimmung und die Bestimmung der arteriellen - O₂ Sättigung erfolgen kann. Für die vorliegende Anwendung wird hauptsächlich nicht die O₂-Sättigung, sondern der dynamische Verlauf des Signals des Detektors verarbeitet. Selbstverständlich kann auch die O₂-Sättigung zur Beeinflussung von Parametern des Magnetfeldes verwendet werden. Im Vordergrund steht hier jedoch die Parameter-Regelung durch das erfaßte Pulssignal.

Wesentlichen Einfluß auf die Herzratenvariabilität haben die beiden Herznerven Sympatikus und Parasympatikus (nervus vagus). Die beeinflussen die Herzfunktion, wobei die Herzfrequenz durch den Parasympatikus vermindert und durch den Sympatikus erhöht wird.

Bei der Herzratenvariabilitäts-Analyse wird über einen bestimmten Zeitabschnitt der Pulssignale (z.B. 1 oder 2 Minuten) der Herzzyklus ermittelt. Der Herzzyklus ist der Kehrwert der Pulsfrequenz und stellt den zeitlichen Abstand zwischen zwei Pulsschlägen, d.h. zwischen zwei benachbarten Maxima des Signals des Pulssensors dar. In Englisch wird der Herzzyklus als "interbeat interval (IBI)" bezeichnet. Die lückenlose Folge der nebeneinander aufgetragenen Herzzyklen wird als Tachogramm bezeichnet. Zur Ermittlung der Herzratenvariablilität werden die in diesem Tachogramm enthaltenen Frequenzanteile einer Spektralanalyse unterzogen, d.h., es wird für jeden Frequenzanteil die Amplitude ermittelt. Die niederfrequenten Anteile des Frequenzspektrums (LF = low frequency = 0,04 - 0,15 Hz) werden vorwiegend den Einflüssen des Sympatikus zugeordnet. Die hochfrequenten Anteile (HF = high frequency = 0,15-0,5 Hz) werden vorwiegend den Einflüssen des Parasympatikus zugeordnet. Der Quotient aus der LF- und HF-Komponente wird als Indikator für sympatico-vagale Aktivität betrachtet. Die LF-Komponente besteht aus dem Integral der Amplituden im Low Frequency-Bereich von 0,04 bis 0,15 Hz. Die HF-Komponente besteht aus dem Integral der Amplituden im High-Frequency-Bereich zwischen 0,15 und 0,4 Hz. Der Wert des genannten Quotienten liegt unter Normalbedingungen üblicherweise zwischen 1,5 und 2. In der Regel ist es das Ziel, den Patienten in diesem Normalzustand zu versetzen. Überwiegt der Einfluß des Sympatikus, muß der Patient sedierend beeinflußt werden, um den Normalzustand zu erreichen. Überwiegt der Einfluß des Parasympatikus, muß ein tonisierendes Programm gewählt werden, um den Patienten in den Normalzustand zu versetzen.

Die Parameter der Magnetfeldtherapie werden entsprechend dem ermittelten Herzratenvariabilitäts-Quotienten eingestellt. Ein erster Parametersatz ist beispielsweise einer tonisierenden Magnetfeldtherapie zugeordnet und ein zweiter Parametersatz einer sedierenden. In Abhängigkeit von dem Herzratenvariabilitäts-Quotienten werden die einzelnen Parameter zwischen ihrem jeweiligen Wert aus dem ersten Parametersatz und ihrem jeweiligen Wert aus dem zweiten Parametersatz interpoliert. Der Herzratenvariabilitäts-Quotient kann zum Zwecke der Interpolation derart skaliert und normiert werden, daß er in einem Bereich zwischen 0 und 1 liegt, wobei der Wert 0 beispielsweise dem sedierenden Programm und der Wert 1 dem tonisierenden Programm zugeordnet ist.

Das gewählte Beispiel der Parametersteuerung zwischen zwei konkreten Parametersätzen läßt sich selbstverständlich erweitern. Beispielsweise können mehrere Parametersätze mit verschieden stark tonisierenden oder sedierenden Einflüssen und ggf. weiteren therapeutischen Wirkungen verwendet werden, wobei aufgrund der dem Pulsverlauf entnommenen Kenngrößen zwischen diesen mehreren Parametersätzen ausgewählt und erforderlichenfalls interpoliert wird. Auch kann neben dem oben beschriebenen Herzratenvariabilitäts-Quotienten ein anderer oder ein zusätzlicher Indikator aus dem Meßsignal des Pulssensors ermittelt werden, mit dem die Parameter zur Magnetfelderzeugung beeinflußt werden. Bei Verwendung eines Puls-Oximetersensors kann - wie bereits erwähnt - auch der Sauerstoffgehalt des Blutes analysiert und als Indikator für die Einstellung der Parameter des Magnetfeldes verwendet werden.

Die erfindungsgemäße Anordnung weist in einer praktischen Ausführungsform zur Durchführung des erfmdungsgemäßen Verfahrens einen Ringspeicher auf, in dem ausgehend von den aktuellste Meßsignalen der zeitliche Pulsverlauf über einen bestimmten vorangehenden Zeitraum gespeichert wird. In dem Ringspeicher wird somit ausgehend von aktuellen Zeitpunkt ein Zeitabschnitt der vorangehenden Zeit, beispielsweise 1 Minute des Pulsverlaufs, abgespeichert. Aus diesem gespeicherten Signal wird, wie oben beschrieben., der Herzratenvariabilitäts-Quotient ermittelt, der für die Herzzyklusschwankungen repräsentativ ist.

Wie oben erwährt, ist die Pulssignalauswertung nur eine von mehreren Möglichkeiten, die Magnetfeldtherapie mittels eines Bioparameters zu regeln. Alternativ oder zusätzlich können andere Kenngrößen und Merkmale des aufgenommenen Signals verwendet werden, beispielsweise die Atmungsformen, Sauerstoffsättigung des Blutes sowie weitere bekanntermaßen aus den oben genannten Bioparametern abgeleitete Kenngrößen.

Die erfindungsgemäße Regelanordnung kann selbstverständlich nicht nur ein Magnetfeldtherapiegerät, sondern ein zusätzliches Therapiegerät regeln, welches an das gleiche Steuergerät angeschlossen ist. Als zusätzliche Therapiegeräte eignen sich beispielsweise Klangerzeugungsmittel für die Ton- und Klangtherapie, Lichterzeugungsmittel für Farb- und Lichttherapie, Elektroden für eine Elektrostimulationstherapie, Geräte zur Erzeugung elektrischer Wechselfelder (Frequenztherapiegeräte), Schwingungstherapiegeräte, welche mechanische Schwingungen erzeugen, Wärmestrahler für die Wärmetherapie, Sauerstofftherapiegeräte.

Nachfolgend wird eine Ausführungsform der Erfindung in Verbindung mit den beigefügten Zeichnungen beschrieben. In den beigefügten Zeichnungen zeigen:
- **Fig. 1**: eine schaubildliche Darstellung der erfindungsgemäßen Therapieanordnung;
- **Fig. 2**: ein Diagramm, in dem die einzelnen Bestandteile der Anordnung dargestellt sind;
- **Fig. 3** ein: Ablaufdiagramm des erfindungsgmäßen Verfahrens;
- **Fig. 4**: eine schematische Darstellung einer gemessenen Pulskurve;
- **Fig. 5**: eine aus der Pulskurve abgeleitete Herzzyklusreihe;
- **Fig. 6**: eine bereinigte Herzzyklusreihe, in der Artefakte, d.h. künstlich Einflüsse auf das Meßsignal, herausgefiltert sind und
- **Fig. 7**: das Ergebnis einer Spektralanalyse des Herzzyklussignals.

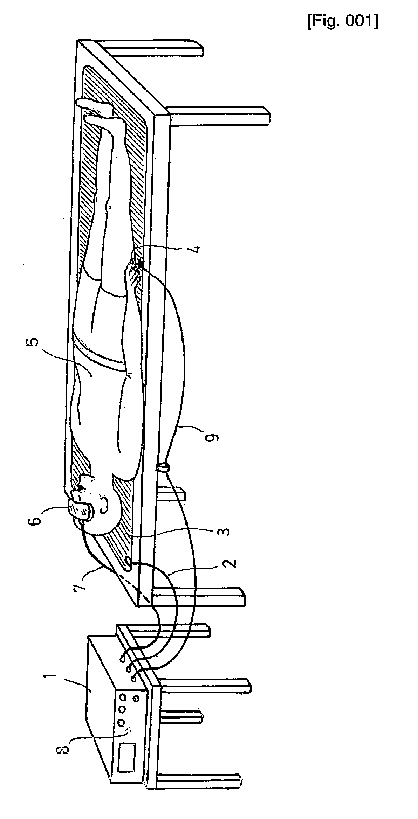

Die Fig. 1 zeigt die erfindungsgemäße Magnetfeldtherapieanordnung mit einem Steuergerät 1, an welches über ein Anschlußkabel 2 eine Magnetfeldmatte 3 angeschlossen ist. In der Magnetfeldmatte 3 verläuft eine Anzahl elektrischer Leiterbahnen, die elektrisch leitend über das Anschlußkabel 2 mit dem Steuergerät 1 verbunden sind. Über das Steuergerät 1 wird ein Strom in die elektrischen Leiterbahnen der Magnetfeldmatte 3 eingeleitet, der ein Magnetfeld über der Magnetfeldmatte 3 erzeugt. In einer praktischen Ausführungsform ist der Strom zeitlich variabel und verläuft in Einzelimpulsen, welche in Impulsgruppen zusammengefaßt sind, die jeweils durch Pausen zwischen zwei Impulsgruppen getrennt sind. Die Form und Frequenz der einzelnen Stromimpulse, der zeitlich variable Amplitudenverlauf der Stromimpulse sowie die Pausen zwischen den aufeinanderfolgenden Impulsgruppen (der Kehrwert der Periode der Impulsgruppen wird Burst-Frequenz genannt) haben wesentlichen Einfluß auf die Wirkung des Magnetfeldes auf den Organismus des Patienten. Üblicherweise wird an dem Steuergerät ein fester Wert für diese Parameter eingegeben oder ein zuvor definierter Ablauf dieser Parameter ausgewählt, um eine bestimmte Therapiewirkung zu erzielen.

Bei der erfindungsgemäßen Anordnung ist ein Fingersensor 4 vorgesehen, der dazu dient, den Puls des Patienten 5 repräsentierende Meßsignale aufzunehmen und dem Steuergerät 1 zuzuführen. Das Steuergerät 1 kann aufgrund dieser Pulssignale einen oder mehrere Indikatoren ermitteln, mit denen die Parameter des Magnetfeldes geregelt werden.

In Fig. 1 ist ein weiteres Therapiegerät 6 in Form einer Farbtherapiebrille gezeigt, welche die Augen des Patienten 5 gegen äußere Lichteinflüsse abschirmt und in der verschiedenfarbiges Licht in Impulsen und mit Farbwechseln erzeugt wird, um die therapeutische Wirkung der Magnetfeldmatte 3 zu unterstützen. Die Farbtherapiebrille 6 ist ebenfalls durch ein Anschlußkabel 7 mit dem Steuergerät verbunden. Bei autonomer Stromversorgung können die Therapiegeräte (Magnetfeldmatte 3 und Farbtherapiebrille 6) auch über kabellose Datenverbindung (z.B. Bluetooth oder Wireless LAN) durch das Steuergerät 1 angesteuert werden.

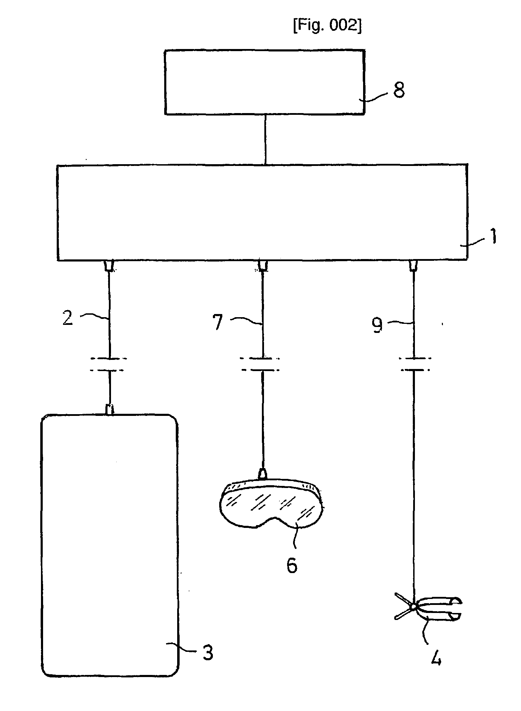

Die Fig. 2 zeigt die Komponente der erfindungsgemäßen Therapieanordnung. Kernstück ist das Steuergerät 1, welches entweder an seiner Vorderseite oder Oberseite ein Steuerpult S aufweist oder über eine Datenverbindung mit einem derartigen Steuerpult 8 verbunden ist. Das Steuerpult 8 ist mit Schaltern und Tasten zur Einstellung des Steuergerätes 1 versehen. Ferner weist es analoge oder digitale Anzeigevorrichtungen auf, auf welchen die Einstellungen des Steuergerätes 1 darstellbar sind.

Die Magnetfeldmatte 3, welche die Applikationsvorrichtung der Therapieanordnung bildet, ist über ein Anschlußkabel 2 mit dem Steuergerät 1 verbunden. Das Steuergerät 1 generiert einen bestimmten Stromverlauf, dessen Parameter je nach gewünschter Therapiewirkung eingestellt werden können. Der Strom wird durch die Leiterbahnen der Magnetfeldmatte 3 geleitet, so daß um diese Leiterbahnen ein Magnetfeld aufgebaut wird, dessen Parameter direkt von den Parametern des eingeleiteten Stroms abhängig sind.

Ein weiteres Therapiegerät 6, z.B. die in Fig. 1 dargestellte Farbtherapiebrille, ist über das zweite Anschlußkabel 7 mit dem Steuergerät verbunden und empfängt ebenfalls Steuerströme zur Erzeugung der Therapiewirkung des Therapiegerätes 6.

Zur Erfassung des Pulses des Patienten 5 ist an das Steuergerät 1 ein Puls-Oximetersensor 4, nämlich ein Fingersensor, angeschlossen, der in der oben beschriebene Weise funktioniert. Über das Signalkabel 9, welches den Puls-Oximetersensor 4 mit dem Steuergerät 1 verbindet, wird zum einen die Speisespannung für die Lichtquelle des Puls-Oximetersensors 4 zugeführt und zum anderen die Meßsignale von dem Detektor des Puls-Oximetersensors 4 an das Steuergerät 1 weitergeleitet. Aufgrund der dem Pulssignal entnommenen Indikatoren regelt das Steuergerät 1 die Parameter des Stroms, welcher der Magnetfeldmatte 3 zugeführt wird.

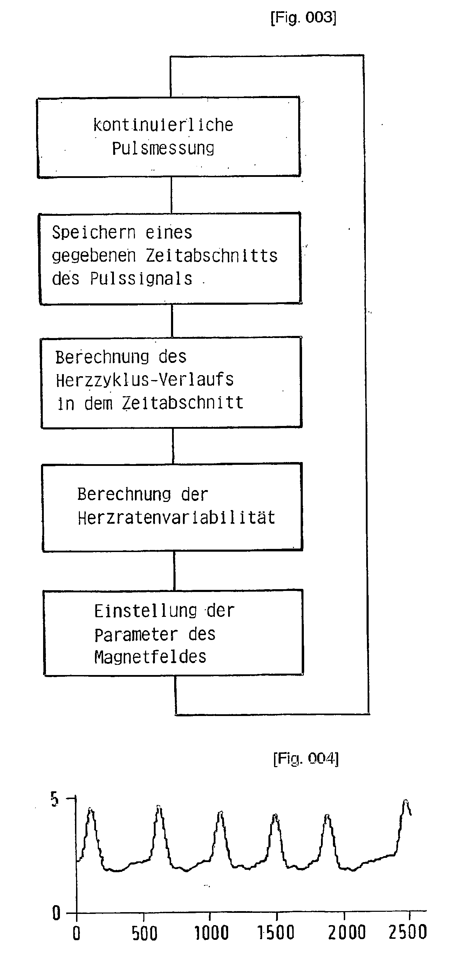

Ein Ablaufdiagramm dieses Regelvorgangs ist in Fig. 3 dargestellt. Die Meßsignale der kontinuierlichen Pulsmessung des Puls-Oximetersensors 4 werden abgespeichert. Hierfür steht ein Ringspeicher zur Verfügung, der jeweils, ausgehend von den jüngsten Meßsignalen, einen vorgegebenen Zeitabschnitt abspeichert. Die jeweils ältesten gespeicherten Signale werden dabei von den jeweils jüngsten Signalen überschrieben, so daß zu jedem Zeitpunkt der gleiche Zeitabschnitt, ausgehend von dem jüngsten Meßsignal, abgespeichert ist. Eine grafische Darstellung des erfaßten Pulssignals ist der Fig. 4 zu entnehmen.

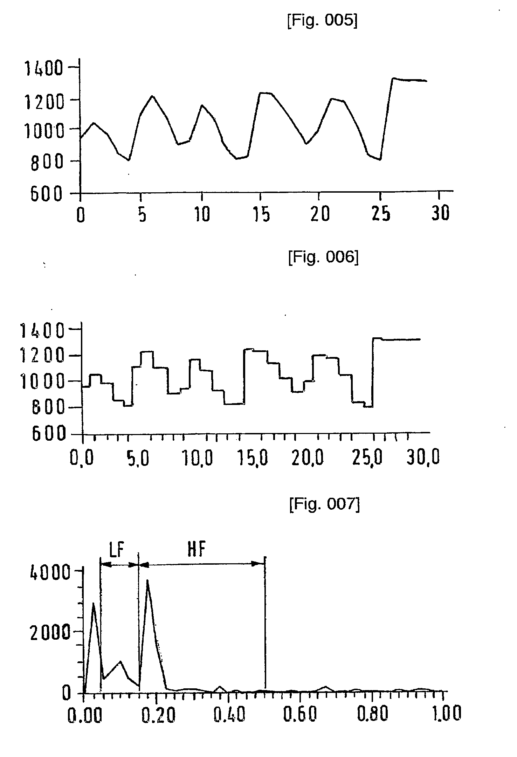

Aus dem Meßsignal des Pulssensors wird der Verlauf des Herzzyklus berechnet. Der Herzzyklus (inter beat interval) ist definiert als Abstand zwischen zwei aufeinanderfolgende Maxima der Pulskurve und stellt die Zeit zwischen zwei Herzschlägen dar. Die Abfolge der aufeinanderfolgenden Herzzyklen, wie sie aus einer Pulskurve errechnet wurde, sind in Fig. 5 als Tachogramm dargestellt. Dieses Tachogramm wird einer Artefaktkorrektur unterzogen, so daß sich ein Tachogramm als äquidistante Zeitreihe ergibt, in der für jeden Herzschlag der vorangehende Herzzyklus abgebildet ist (vgl. Fig. 6).

Das Tachogramm der Fig. 6 wird einer Spektralanalyse oder Frequenzanalyse unterzogen, wobei für die verschiedenen Frequenzanteile des Tachogramms die jeweilige Amplitude dargestellt wird. Derartige Analyseverfahren sind im Stand der Technik bekannt. Als Beispiel sei die Fast-Fourrier-Transformation erwähnt.

Das Ergebnis der Spektralanalyse ist in Fig. 7 gezeigt. Wie oben beschrieben, kann ein Herzratenvariabilitäts-Quotient definiert werden, der sich aus dem Quotienten zwischen niederfrequenter Komponente und hochfrequenter Komponente der Spektralanalyse ergibt. Die niederfrequente Komponente (LF = low frequency) errechnet sich als das Integral der Amplituden im Bereich zwischen 0,04 und 0,15 Hz. Die hochfrequente Komponente (HL = high frequency) errechnet sich als das Integral der Amplituden im Intervall zwischen 0,15 und 0,4 Hz. Dieser Quotient wird zur Steuerung des Therapiegerätes herangezogen. Genaue Interpretationen der Aussagen und Informationen über die Meßverfahren finden sich in der Fachliteratur, beispielsweise in den Veröffentlichungen der Task Force of the European Society of Cardiology and the North American Society of Pacing and Electrophysiology: Heart rate variability. Standards of measurement, physiological Interpretation, and clinical use. Circulation 1996 (93) 1043-1065.

Die aufgenommenen Meßsignale können natürlich auch zur Steuerung der anderen Therapiegeräte, beispielsweise der Farbtherapiebrille 6, verwendet werden. Auch können ggf. andere Indikatoren aus den gemessenen Signalen abgeleitet werden als die genannte Herzratenvariabilität.

### Bezugszeichenliste:

- 1: Steuergerät
- 2: Anschlußkabel
- 3: Applikationsmittel, Magnetfeldmatte
- 4: Puls-Oximetersensor, Fingersensor
- 5: Patient
- 6: Farbtherapiebrille, Farbtherapiegerät
- 7: Anschlußkabel
- 8: Steuerpult
- 9: Signalkabel

## Patentansprüche

1. Anordnung zur Durchführung einer Magnetfeldtherapie, mit
- einem Applikationsmittel (3) zum Aufbringen eines Magnetfeldes auf ein Lebewesen (5),
- einem Steuergerät (1) zur Einstellung mindestens eines Parameters des Magnetfeldes,
- einem Pulssensor (4) zur Erfassung des Pulses des Lebewesens (5) und
- einer Regelanordnung zur Einstellung des genannten Parameters in Abhängigkeit von den Meßsignalen des Pulssensors (4),
**gekennzeichnet durch** einen Schaltkreis zur Ermittlung der Herzratenvariabilität aus den Meßsignalen des Pulssensors (4), wobei die Regelanordnung den genannten Parameter in Abhängigkeit von der ermittelten Herzraten variabilität einstellen kann.

2. Anordnung nach Anspruch 1, **dadurch gekennzeichnet, daß** sie einen zusätzlichen Biosensor zum Erfassen mindestens einer der folgenden vegetativen oder motorischen Funktionen des Lebewesens (5) aufweist: Blutdruck, Sauerstoffsättigung des Blutes, Aktionspotentiale des Herzens (Elektrokardiogramm), Potentialschwankungen des Gehirns (Elektroenzephalogramm), Hauttemperatur, Hautwiderstand, Atemfrequenz, Atemvolumen, Atemgaszusammensetzung.

3. Anordnung nach Anspruch 1 oder 2, **dadurch gekennzeichnet, daß** der zusätzliche Biosensor mindestens einer der folgenden Biosensoren ist: Meßelektrode, Temperatursensoren, Widerstandssensoren, Atemmeßgerät, Atemgasanalysegerät.

4. Anordnung nach einem der vorangehenden Ansprüche, **dadurch gekennzeichnet, daß** das Steuergerät (1) und die Regelanordnung elektrische Schaltungen zur Einstellung mehrerer Parameter des Magnetfeldes aufweisen.

5. Anordnung nach einem der vorangehenden Ansprüche, **dadurch gekennzeichnet, daß** der genannte Parameter ein Parameter eines Stromsignals ist, mit welchem ein elektrische Leiter des Applikationsmittels (3) beaufschlagt wird.

6. Anordnung nach Anspruch 5, **dadurch gekennzeichnet, daß** der oder die genannten Parameter aus der folgenden Gruppe ausgewählt sind: Dauer eines Stromimpulses, Frequenz innerhalb einer Gruppe von Stromimpulsen, Zeitabstand zwischen zwei aufeinanderfolgenden Stromimpulsgruppen, Stromstärke, Spannung.

7. Anordnung nach einem der vorangehenden Ansprüche, **dadurch gekennzeichnet, daß** der Pulssensor ein Puls-Oximetersensor (4), zum Beispiel ein Fingersensor oder Ohrklemmensensor, ist.

8. Anordnung nach einem der vorangehenden Ansprüche, **dadurch gekennzeichnet, daß** der Schaltkreis einen Speicher zum Erfassen eines Abschnittes zeitlichen Pulsverlaufs aufweist.

9. Anordnung nach Anspruch 8, **dadurch gekennzeichnet, daß** der Speicher als Ringspeicher ausgebildet ist, in dem ausgehend von den aktuellste Meßsignalen der zeitliche Pulsverlauf eines bestimmten vorangehenden Zeitraums gespeichert wird.

10. Anordnung nach einem der Ansprüche 8 und 9, **dadurch gekennzeichnet, daß** der Schaltkreis eine Baugruppe zur Ermittlung des zeitlichen Verlaufs des Herzzyklus und eine Baugruppe zur Ermittlung der Herzzyklusschwankungen aus dem zeitlichen Pulsverlauf aufweist.

11. Anordnung nach einem der vorangehenden Ansprüche, **dadurch gekennzeichnet, daß** sie mindestens ein zusätzliches Therapiegerät (6) umfaßt, welches an das Steuergerät (1) angeschlossen ist.

12. Anordnung nach Anspruch 11, **dadurch gekennzeichnet, daß** das zusätzliche Therapiegerät (6) ausgewählt ist aus der Gruppe folgender Therapiegeräte: Elektrostimulationsgeräte, Ton- und Klangtherapiegeräte, Lichttherapiegeräte, Farbtherapiegeräte (6), Frequenztherapiegeräte, Schwingungstherapiegeräte, Wärmetherapiegeräte, Sauerstofftherapiegeräte.

13. Verfahren zur Bereitstellung eines zeitlich variierenden Stroms durch ein Steuergerät (1) für die Erzeugung eines Magnetfeldes, wobei der Strom an ein Applikationsmittel (3) geleitet wird, welches zur Durchführung einer Magnetfeldtherapie das Magnetfeld auf ein Lebewesen (5) aufbringen kann, wobei der Puls des Lebewesens (5) mittels eines Pulssensors (4) erfaßt wird, **dadurch gekennzeichnet, daß** die Herzratenvariabilität aus den Meßsignalen des Pulssensors (4) ermittelt wird und der Stromverlauf durch das Steuergerät (1) in Abhängigkeit von der ermittelten Herzratenvariabilität eingestellt wird.

14. Verfahren nach Anspruch 13, **dadurch gekennzeichnet, daß** zusätzlich mindestens eine der folgenden vegetativen oder motorischen Funktion des Lebewesens (5) erfaßt und zum Einstellen des Stromverlaufs verwendet wird: Blutdruck, Sauerstoffsättigung des Blutes, Aktionspotentiale des Herzens (Elektrokardiogramm), Potoentialschwankungen des Gehirns (Elektroenzephalogramm), Hauttemperatur, Hautwiderstand, Atemfrequenz, Atemvolumen, Atemgaszusammensetsung.

15. Verfahren nach Anspruch 13 oder 14, **dadurch gekennzeichnet, daß** das Steuergerät (1) mehrere Parameter des Stromverlaufs einstellt.

16. Verfahren nach Anspruch 15, **dadurch gekennzeichnet, daß** das Steuergerät (1) mindestens einen Parameter aus der folgenden Gruppe einstellt: Dauer eines Stromimpulses, Frequenz innerhalb einer Gruppe von Stromimpulsen, Zeitabstand zwischen zwei aufeinanderfolgenden Stromimpulsgruppen, Stromstärke, Spannung.

17. Verfahren nach einem der Ansprüche 13 bis 16, **dadurch gekennzeichnet, daß** als Biosensor ein Puls-Oximetersensor (4) verwendet wird.

18. Verfahren nach Anspruch 17, **dadurch gekennzeichnet, daß** ein Fingersensor oder Ohrklemmensensor verwendet wird.

19. Verfahren nach einem der Ansprüche 13 bis 18, **dadurch gekennzeichnet, daß**
- ein Abschnitt des zeitlichen Pulsverlaufs in einem Speicher abgespeichert wird,
- der zeitliche Verlauf des Herzzyklus aus dem gespeicherten Abschnitt des zeitlichen Pulsverlaufs ermittelt wird und
- die Herzzyklusschwankungen aus dem gespeicherten Abschnitt des zeitlichen Pulsverlaufs ermittelt werden.

20. Verfahren nach Anspruch 19, **dadurch gekennzeichnet, daß** in einem Ringspeicher ausgehend von den aktuellsten Meßsignale der zeitliche Pulsverlauf eines bestimmten vorangehenden Zeitraums gespeichert wird.

21. Verfahren nach Anspruch 19 oder 20, **dadurch gekennzeichnet, daß** die Herzzyklusschwankungen durch eine Frequenzanalyse des zeitlichen Verlaufs des Herzzyklus ermittelt werden.

22. Verfahren nach einem der Ansprüche 13 bis 21, **dadurch gekennzeichnet, daß** durch das Steuergerät (1) aufgrund der Meßsignale des Biosensors (4) ein Satz mit mehreren Parametern eingestellt wird, wobei für jeden der Parameter mindestens zwei Stützpunkte festgelegt sind und in Abhängigkeit von den Meßsignalen des Pulssensors (4) zwischen den Stützpunkten interpoliert wird.

23. Verfahren nach einem der Ansprüche 13 bis 22, **dadurch gekennzeichnet, daß** mindestens ein zusätzliches durch das Steuergerät (1) gesteuertes Therapiegerät (6) auf das Lebewesen (5) einwirkt.

24. Verfahren nach Anspruch 23, **dadurch gekennzeichnet, daß** das zusätzliche Therapiegerät ausgewählt ist aus der folgenden Gruppe:Elektrostimulationsgeräte, Ton- und Klangtherapiegeräte, Lichttherapiegeräte; Farbtherapiegeräte (6), Frequenztberapiegeräte, Schwingungsthernpieäte, Wärmetherapiegeräte, Sauerstofftherapiegeräte.

## Claims

1. Assembly for carrying out magnetic field therapy, comprising
- an application means (3) for applying a magnetic field to a living being (5),
- a control unit (1) for adjusting at least one parameter of the magnetic field,
- a pulse sensor (4) for recording the pulse of the living being (5) and
- a regulating assembly for adjusting said parameter In response to the measuring signals of the pulse sensor (4),
**characterized by** a circuit for determining the heart rate variability from the measuring signals of the pulse sensor (4), wherein the regulating assembly is capable of adjusting said parameter In response to the heart rate variability determined.

2. Assembly according to claim 1, **characterized in that** it comprises an additional biosensor for recording at least one of the following vegetative or motoric functions of the living being (5):
blood pressure, oxygen saturation of the blood, action potentials In the heart (electrocardiogram), potential fluctuations in the brain (electroencephalogram), skin temperature, skin resistance, respiratory rate, respiratory volume, respiratory gas composition.

3. Assembly according to claim 1 or 2, **characterized in that** the additional biosensor is at least one of the following biosensors:
measuring electrodes, temperature sensors, resistance sensors, respiratory measuring device, respiratory gas analysis device.

4. Assembly according to one of the preceding claims, **characterized in that** the control unit (1) and the regulating assembly have electrical circuits for adjusting several parameters of the magnetic field.

5. Assembly according to one of the preceding claims, **characterized in that** said parameter Is a parameter of a current signal which is applied to an electrical conductor in the application means (3).

6. Assembly according to claim 5, **characterized in that** said parameter or parameters are selected from the following group: duration of a current pulse, frequency within a group of current pulses, time interval between two successive current pulse groups, current intensity, voltage.

7. Assembly according to one of the preceding claims, **characterized in that** the pulse sensor is a pulse oximeter sensor (4), for example a finger sensor or an ear clip sensor.

8. Assembly according to one of the preceding claims, **characterized in that** the circuit has a memory for recording a segment of a temporal pulse path.

9. Assembly according to claim 8, **characterized in that** the memory is a circular buffer memory In which based on the most up-to-date measuring signals, the temporal pulse pattern of a specific preceding period is stored.

10. Assembly according to one of claims 8 and 9, **characterized in that** the circuit has a component for determining the temporal course of the cardiac cycle and a component for determining the cardiac cycle fluctuations from the temporal pulse pattern.

11. Assembly according to one of the preceding claims, **characterized in that** it comprises at least one additional therapeutic device (6) which is connected to the control unit (1).

12. Assembly according to claim 11, **characterized in that** the additional therapeutic device (6) is selected from the following group of therapeutic devices: electrostimulation devices, audio and sound therapy devices, light therapy devices, colour therapy devices (6), frequency therapy devices, vibration therapy devices, thermal therapy devices, oxygen therapy devices.

13. Method for providing a time-variant current by means of a control unit (1) for generating a magnetic field, In which the current is supplied to an application means (3), which, for the purpose of carrying out a magnetic field therapy, can apply the magnetic field to a living being (5), wherein the pulse of the living being (5) is recorded by means of a pulse sensor (4), **characterized in that** the heart rate variability is determined from the measuring signals of the pulse sensor (4) and the current flow through the control unit (1) is set In response to the determined heart rate variability.

14. Method according to claim 13, **characterized in that** in addition at least one of the following vegetative or motoric functions of the living being (5) is recorded and used to set the current flow: blood pressure, oxygen saturation of the blood, action potentials In the heart (electrocardiogram), potential fluctuations in the brain (electroencephalogram), skin temperature, skin resistance, respiratory rate, respiratory volume, respiratory gas composition.

15. Method according to claim 13 or 14, **characterized in that** the control unit (1) sets several parameters of the current flow.

16. Method according to claim 15, **characterized in that** the control unit (1) sets at least one parameter from the following group: duration of a current pulse, frequency within a group of current pulses, time interval between two successive current pulse groups, current intensity, voltage.

17. Method according to one of claims 13 to 16, **characterized in that** a pulse oximeter sensor (4) is used as a biosensor.

18. Method according to claim 17, **characterized in that** a finger sensor or ear clip sensor is used.

19. Method according to one of claims 13 to 18, **characterized in that**
- one segment of the temporal pulse pattern Is stored In a memory,
- the temporal pattern of the cardiac cycle is determined from the stored segment of the temporal pulse pattern, and
- the cardiac cycle fluctuations are determined from the stored segment of the temporal pulse pattern.

20. Method according to claim 19, **characterized in that** based on the most up-to-date measuring signals, the temporal pulse pattern corresponding to a specific preceding time period is stored in a circular buffer memory.

21. Method according to claim 19 or 20, **characterized in that** the cardiac cycle fluctuations are determined by a frequency analysis of the temporal course of the cardiac cycle.

22. Method according to one of claims 13 to 21, **characterized in that** the control unit (1) uses the measuring signals from the biosensor (4) to establish a set with several parameters, wherein at least two support points are defined for each parameter and interpolation between the support points Is performed In response to the measuring signals of the pulse sensor (4).

23. Method according to one of claims 13 to 22, **characterized in that** at least one additional therapeutic device (6) controlled by the control unit (1) acts on the living being (5).

24. Method according to claim 23, **characterized in that** the additional therapeutic device Is selected from the following group: electrostimulation devices, audio and sound therapy devices, light therapy devices, colour therapy devices (6), frequency therapy devices, vibration therapy devices, thermal therapy devices, oxygen therapy devices.

## Revendications

1. Agencement destiné à mettre en ouvre une magnétothérapie, avec
- un moyen d'application (3) pour l'application d'un champ magnétique sur un être vivent (5),
- un appareil de commande (1) pour un ajustement d'au moins un paramètre du champ magnétique,
- un capteur de pouls (4) pour un enregistrement du pouls de l'être vivant (5) et
- un agencement de régulation pour un ajustement du paramètre mentionné en fonction des signaux de mesure du capteur de pouls (4),
**caractérisé par** un circuit de détermination de la variabilité de la fréquence cardiaque à partir des signaux de mesure du capteur de pouls (4), l'agencement de régulation pouvant ajuster le paramètre mentionné en fonction de la variabilité déterminée de la fréquence cardiaque.

2. Agencement selon la revendication 1, **caractérisé en ce qu'**il présente un blocapteur supplémentaire pour l'enregistrement d'au moins une des fonctions végétatives ou motrices suivantes de l'être vivant (5) :
tension artérielle, saturation du sang en oxygène, potentiels d'action cardiaques (électrocardiogramme), variations du potentiel cérébral (électroencéphalogramme), température de la peau, résistance électrique de la peau, fréquence respiratoire, volume respiratoire, composition du gaz respiratoire.

3. Agencement selon la revendication 1 ou 2, **caractérisé en ce que** le biocapteur supplémentaire est au moins un des deux capteurs suivants :
électrodes de mesure, capteurs de température, capteurs de résistance, appareil de mesure du souffle, appareil d'analyse du gaz respiratoire.

4. Agencement selon l'une quelconque des revendications précédentes, **caractérisé en ce que** l'appareil de commande (1) et l'agencement de régulation présentent des connexions électriques en vue de l'ajustement de plusieurs paramètres du champ magnétique.

5. Agencement selon l'une quelconque des revendications précédentes, **caractérisé en ce que** le paramètre mentionné est un paramètre d'un signal de courant, avec lequel un conducteur électrique du moyen d'application (3) est sollicité.

6. Agencement selon la revendication 5, **caractérisé en ce que** le ou les paramètre(s) mentionné(s) sont choisis parmi le groupe suivant ; durée d'une impulsion de courant, fréquence à l'intérieur d'un groupe d'impulsions de courant, écart de temps entre deux groupes d'impulsions de courant successifs, intensité, tension.

7. Agencement selon l'une quelconque des revendications précédentes, **caractérisé en ce que** le capteur de pouls est un capteur oxymétrique de pouls (4), par exemple un capteur à mettre en place sur un doigt ou un capteur formant oreillette.

8. Agencement selon l'une quelconque des revendications précédentes, **caractérisé en ce que** le circuit présente une mémoire pour l'enregistrement d'une partie d'un déroulement temporel de pouls.

9. Agencement selon la revendication 8, **caractérisé en ce que** la mémoire est conçue en tant que mémoire tampon circulaire, dans laquelle le déroulement temporel de pouls d'une période précédente déterminée est mémorisé en partant des signaux de mesure les plus pertinents.

10. Agencement selon l'une quelconque des revendications 8 et 9, **caractérisé en ce que** le circuit présente un élément de détermination du déroulement temporel du cycle cardiaque et un élément de détermination des variations de cycle cardiaque à partir du déroulement temporel de pouls.

11. Agencement selon l'une quelconque des revendications précédentes, **caractérisé en ce qu'**il comprend au moins un appareil de thérapie (6) supplémentaire, qui est raccordé à l'appareil de commande (1).

12. Agencement selon la revendication 11, **caractérisé en ce que** l'appareil de thérapie (6) supplémentaire est sélectionné parmi le groupe constitué des appareils de thérapie suivants : appareils d'électrostimulatlon, appareils de musicothérapie, appareils de luminothérapie, appareils (6) de chromothérapie, appareils de thérapie interférentielle, appareils de thérapie utilisant des vibrations, appareils de thermothérapie, appareils d'oxygénothérapie.

13. Procédé de préparation d'un courant variant dans le temps grâce à un appareil de commande (1) pour la production d'un champ magnétique, le courant étant conduit au niveau d'un moyen d'application (3) qui peut appliquer le champ magnétique sur un être vivant pour la mise en ouvre d'une magnétothérapie, le pouls de l'être vivant (5) étant enregistré au moyen d'un capteur de pouls (4), **caractérisé en ce que** la variabilité de la fréquence cardiaque est déterminée à partir des signaux de mesure du capteur de pouls (4) et l'écoulement du courant est ajusté par l'appareil de commande (1) en fonction de la variabilité déterminée de la fréquence cardiaque.

14. Procédé selon la revendication 13, **caractérisé en ce qu'**au moins une des fonctions végétatives ou motrices suivantes de l'être vivant (5) est enregistrée de manière supplémentaire et est utilisée pour l'ajustement de l'écoulement de courant: tension artérielle, saturation du sang en oxygène, potentiels d'action cardiaques (électrocardiogramme), variations du potentiel cérébral (éloctroencéphalogramme), température de la peau, résistance électrique de la peau, fréquence respiratoire, volume respiratoire, composition du gaz respiratoire.

15. Procédé selon la revendication 13 ou 14, **caractérisé en ce que** l'appareil de commande (1) ajuste plusieurs paramètres de l'écoulement de courant.

16. Procédé selon la revendication 15, **caractérisé en ce que** l'appareil de commande (1) ajuste au moins un paramètre issu du groupe suivant : durée d'une impulsion de courant, fréquence à l'intérieur d'un groupe d'impulsions de courant, écart de temps entre deux groupes d'impulsions de courant successifs, intensité, tension.

17. Procédé selon l'une quelconque des revendications 13 à 16, **caractérisé en ce qu'**un capteur oxymétrique de pouls (4) est utilisé en tant que blocapteur.

18. Procédé selon la revendication 17, **caractérisé en ce qu'**un capteur à mettre en place sur un doigt ou un capteur formant oreillette est utilisé.

19. Procédé selon l'une quelconque des revendications 13 à 18, **caractérisé en ce que**
- une partie du déroulement temporel de pouls est mémorisée dans une mémoire,
- le déroulement temporel du cycle cardiaque est déterminé à partir de la partie mémorisée du déroulement temporel de pouls et
- les variations de cycle cardiaque sont déterminées à partir de la partie mémorisée du déroulement temporel de pouls.

20. Procédé selon la revendication 19, **caractérisé en ce que** le déroulement temporel de pouls d'une période précédente déterminée est mémorisé dans une mémoire tampon circulaire en partant des signaux de mesure les plus pertinents.

21. Procédé selon la revendication 19 ou 20, **caractérisé en ce que** les variations de cycle cardiaque sont déterminées grâce à une analyse fréquentielle du déroulement temporel du cycle cardiaque.

22. Procédé selon l'une quelconque des revendications 13 à 21, **caractérisé en ce qu'**une expression comportant plusieurs paramètres est ajustée grâce à l'appareil de commande (1) en fonction des signaux de mesure du biocapteur (4), au moins deux points de base étant définis pour chacun des paramètres et une interpolation ayant lieu entre les points de base en fonction des signaux de mesure du capteur de pouls (4).

23. Procédé selon l'une quelconque des revendications 13 à 22, **caractérisé en ce qu'**au moins un appareil de thérapie (6) supplémentaire commandé par l'appareil de commande (1) influe sur l'être vivant (5).

24. Procédé selon la revendication 23, **caractérisé en ce que** l'appareil de thérapie supplémentaire est sélectionné parmi le groupe suivant : appareils d'électrostimulatlon, appareils de musicothérapie, appareils de luminothérapie, appareils (6) de chromothérapie, appareils de thérapie interférentielle, appareils de thérapie utilisant des vibrations, appareils de thermothérapie, appareils d'oxygénothérapie.
